(19) Europäisches Patentamt
European Patent Office
Office européen des brevets

(11) Veröffentlichungsnummer: **0 167 491**
**A2**

# (12) EUROPÄISCHE PATENTANMELDUNG

(21) Anmeldenummer: **85810303.9**

(22) Anmeldetag: **01.07.85**

(51) Int. Cl.[4]: **C 07 D 239/66**
C 07 D 401/12, A 61 K 31/5-15
C 07 C 119/048

(30) Priorität: **06.07.84 CH 3288/84**

(43) Veröffentlichungstag der Anmeldung:
**08.01.86 Patentblatt 86/2**

(84) Benannte Vertragsstaaten:
**AT BE CH DE FR IT LI LU NL SE**

(71) Anmelder: **CIBA-GEIGY AG**
**Klybeckstrasse 141**
**CH-4002 Basel(CH)**

(72) Erfinder: **Kühne, Manfred, Dr.**
**Alemannenweg 11**
**CH-4148 Pfeffingen(CH)**

(72) Erfinder: **Gallay, Jean Jacques**
**Blumenrain 19**
**CH-4312 Magden(CH)**

## (54) Thiobarbitursäurederivate.

(57) Neue 5-Phenylcarbamoylthiobarbitursäurederivate der allgemeinen Formel

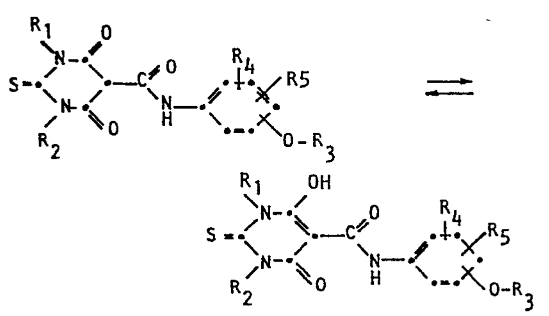

worin

$R_1$ und $R_2$ unabhängig voneinander $C_1$-$C_5$-Alkyl oder Methoxy,

$R_3$ unsubstituiertes Phenyl, unsubstituiertes Pyridyl, oder ein- bis dreifach durch $C_1$-$C_5$-Alkyl, Halogen, Nitro, $C_1$-$C_5$-Halogenalkyl mit 1 bis 5 Halogenatomen oder Cyano-$C_1$-$C_4$-Alkyl substituiertes Phenyl oder ein- bis dreifach durch $C_1$-$C_5$-Alkyl, Halogen, Nitro oder $C_1$-$C_5$-Halogenalkyl mit 1 bis 5 Halogenatomen substituiertes Pyridyl und

$R_4$ und $R_5$ unabhängig voneinander Wasserstoff, Halogen, $C_1$-$C_5$-Alkyl, $C_1$-$C_2$-Halogenalkyl mit 1 bis 5 Halogenatomen oder Nitro bedeuten, einschliesslich ihrer tautomeren Formen und Salze, als anthelmintische Wirkstoffe. Die Wirkstoffe lassen sich als solche oder in Verbindung mit geeigneten Träger- und weiteren Hilfsstoffen zur Bekämpfung von tier-parasitären Helminthen einsetzen.

EP 0 167 491 A2

CIBA-GEIGY AG
Basel (Schweiz)

5-14999/+

## Thiobarbitursäurederivate

Die vorliegende Erfindung betrifft neue 5-Phenylcarbamoylthiobarbitursäurederivate mit anthelmintischer Wirksamkeit, Mittel, die diese Wirkstoffe als Aktivsubstanzen enthalten sowie die Verwendung der Wirkstoffe bzw. der Mittel zur Bekämpfung von Helminthen, insbesondere von Nematoden, Cestoden und Trematoden in Haus- und Nutztieren, vor allem in Säugetieren.

Die Erfindung betrifft ferner die Herstellung der neuen Wirkstoffe sowie der sie enthaltenden Mittel und bei der Herstellung der Wirkstoffe verwendbare Zwischenprodukte.

Die neuen Verbindungen entsprechen der allgemeinen Formel I

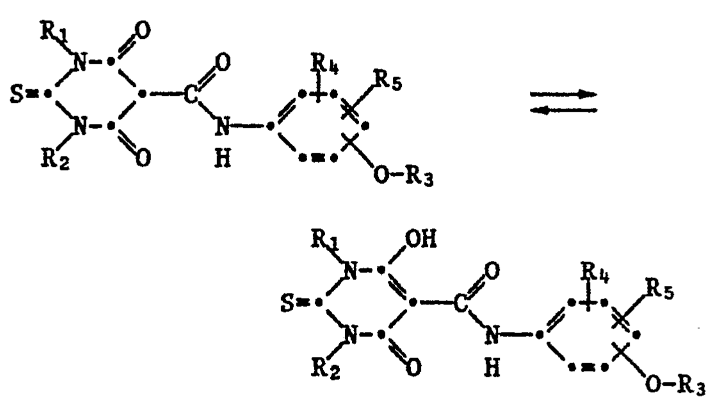

(I)

worin

$R_1$ und $R_2$ unabhängig voneinander $C_1$-$C_5$-Alkyl oder Methoxy,
$R_3$ unsubstituiertes Phenyl, unsubstituiertes Pyridyl, oder ein- bis dreifach durch $C_1$-$C_5$-Alkyl, Halogen, Nitro, $C_1$-$C_5$-Halogenalkyl mit 1 bis 5 Halogenatomen oder Cyano-$C_1$-$C_4$-Alkyl substituiertes Phenyl oder ein- bis dreifach durch $C_1$-$C_5$-Alkyl, Halogen, Nitro oder $C_1$-$C_5$-Halogenalkyl mit 1 bis 5 Halogenatomen substituiertes Pyridyl und
$R_4$ und $R_5$ unabhängig voneinander Wasserstoff, Halogen, $C_1$-$C_5$-Alkyl, $C_1$-$C_2$-Halogenalkyl mit 1 bis 3 Halogenatomen, $C_1$-$C_3$-Alkoxy oder Nitro bedeuten, einschliesslich ihrer tautomeren Formen und Salze.

Bevorzugt sind Verbindungen der Formel I, die in einer der nachfolgend aufgeführten Verbindungsgruppen zusammengefasst sind, und worin

a) $R_1$ und $R_2$ unabhängig voneinander $C_1$-$C_5$-Alkyl oder Methoxy,
$R_3$ unsubstituiertes Phenyl, unsubstituiertes Pyridyl, oder ein- bis dreifach durch $C_1$-$C_5$-Alkyl, Halogen, Nitro, $C_1$-$C_5$-Halogenalkyl mit 1 bis 5 Halogenatomen oder Cyano-$C_1$-$C_4$-Alkyl substituiertes Phenyl oder ein- bis dreifach durch $C_1$-$C_5$-Alkyl, Halogen, Nitro oder $C_1$-$C_5$-Halogenalkyl mit 1 bis 5 Halogenatomen substituiertes Pyridyl und
$R_4$ und $R_5$ unabhängig voneinander Wasserstoff, Halogen, $C_1$-$C_5$-Alkyl, $C_1$-$C_2$-Halogenalkyl mit 1 bis 3 Halogenatomen, oder Nitro bedeuten;

b) $R_1$ Methyl, Aethyl oder Methoxy,
$R_2$ Methyl,
$R_3$ unsubstituiertes Phenyl, unsubstituiertes Pyridyl oder ein- oder zweifach durch $C_1$-$C_5$-Alkyl, Halogen, Nitro, $C_1$-$C_5$-Halogenalkyl mit 1 bis 3 Halogenatomen oder Cyano-$C_1$-$C_4$-Alkyl substituiertes Phenyl oder ein- oder zweifach durch $C_1$-$C_5$-Alkyl, Halogen, Nitro oder $C_1$-$C_5$-Halogenalkyl mit 1 bis 3 Halogenatomen substituiertes Pyridyl und
$R_4$ und $R_5$ unabhängig voneinander Wasserstoff, $C_1$-$C_5$-Alkyl oder Halogen bedeuten;

c) $R_1$ Methyl oder Methoxy,
$R_2$ Methyl,
$R_3$ ein durch Methyl, Chlor, Fluor, Trifluormethyl oder Cyanomethyl substituiertes Phenyl oder ein durch Methyl, Chlor, Fluor oder Trifluormethyl substituiertes Pyridyl und $R_4$ und $R_5$ unabhängig voneinander Wasserstoff, $C_1$-$C_3$-Alkyl, Chlor oder Nitro bedeuten;

d) $R_1$ Methyl oder Methoxy,
$R_2$ Methyl,
$R_3$ ein durch Methyl, Chlor, Trifluormethyl oder Cyanomethyl substituiertes Phenyl oder ein durch Methyl, Chlor oder Trifluormethyl substituiertes Pyridyl und $R_4$ und $R_5$ unabhängig voneinander Wasserstoff oder $C_1$-$C_3$-Alkyl bedeuten und der Rest -O-$R_3$ in 2- oder 4-Stellung steht; und

e) $R_1$ Methyl oder Aethyl,
$R_2$ Methyl,
$R_3$ Phenyl, welches durch Substituenten der Gruppe Fluor, Chlor, Nitro, Trifluormethyl und Cyanomethyl mono- oder disubstituiert ist, oder 2-Pyridyl, welches durch Substituenten der Gruppe Fluor, Chlor, Nitro und Trifluormethyl mono- oder disubstituiert ist, $R_4$ Wasserstoff, Chlor, Methyl, Isopropyl oder Methoxy, und $R_5$ Wasserstoff, Chlor oder Methyl bedeuten, einschliesslich ihrer Triäthylaminsalze.

Von besonderem Interesse sind Verbindungen der Formel I, in denen $R_1$ und $R_2$ jeweils für Methyl, $R_3$ für Phenyl, welches durch Substituenten der Gruppe Chlor und Trifluormethyl mono- oder disubstituiert ist, oder 2-Pyridyl, welches durch Substituenten der Gruppe Chlor und Trifluormethyl mono- oder disubstituiert ist, $R_4$ für Wasserstoff, Methyl, Isopropyl oder Methoxy und $R_5$ für Wasserstoff oder Methyl stehen, wobei der Rest -$OR_3$ in meta- oder para-Position zum Stickstoffatom der Carbamoylgruppe steht, sowie ein Triäthylaminsalz davon, und besonders diejenigen Verbindungen der Formel I, in denen $R_1$ und $R_2$ jeweils für Methyl, $R_3$ für Phenyl, welches durch Substituenten der Gruppe Chlor und Trifluormethyl mono- oder disubstituiert ist, oder 2-Pyridyl, welches durch Substituenten der Gruppe Chlor und Trifluormethyl mono- oder disubstituiert ist, $R_4$ für Wasserstoff oder Methoxy und $R_5$ für Wasserstoff stehen, wobei der Rest $-OR_3$ in meta- oder para-Position zum Stickstoffatom der Carbamoylgruppe steht.

Bevorzugte Einzelverbindungen sind:
1,3-Dimethyl-5-[2-isopropyl-4-(5-trifluormethyl-pyridyl-2-oxy)-phenylcarbamoyl]-2-thiobarbitursäure,
1,3-Dimethyl-5-[4-(5-trifluormethyl-pyridyl-2-oxy)-phenylcarbamoyl]-2-thiobarbitursäure-triäthylaminsalz,
1,3-Dimethyl-5-[4-(3-chlor-5-trifluormethyl-pyridyl-2-oxy)-phenylcarbamoyl]-2-thiobarbitursäure,
1,3-Dimethyl-5-[2,6-dimethyl-4-(5-trifluormethyl-pyridyl-2-oxy)-phenylcarbamoyl]-2-thiobarbitursäure,
1,3-Dimethyl-5-[4-(3,5-dichlor-pyridyl-2-oxy)-phenylcarbamoyl]-2-thiobarbitursäure,
und insbesondere
1,3-Dimethyl-5-[4-(5-trifluormethyl-pyridyl-2-oxy)-phenylcarbamoyl]-2-thiobarbitursäure,
1,3-Dimethyl-5-[4-(4-trifluormethyl-phenoxy)-phenylcarbamoyl]-2-thiobarbitursäure,
1,3-Dimethyl-5-[4-(3-trifluormethyl-phenoxy)-phenylcarbamoyl]-2-thiobarbitursäure,
1,3-Dimethyl-5-[4-methoxy-3-(2-chlor-4-trifluormethyl-phenoxy)-phenylcarbamoyl]-2-thiobarbitursäure,
1,3-Dimethyl-5-[3-methoxy-4-(2-chlor-4-trifluormethyl-phenoxy)-phenylcarbamoyl]-2-thiobarbitursäure und
1,3-Dimethyl-5-[4-methoxy-3-(3,5-dichlor-pyridyl-2-oxy)-phenylcarbamoyl]-2-thiobarbitursäure.

Als Salze der Verbindungen der Formel I kommen in Frage z.B. die Alkalimetall-, Ammonium- oder Aminsalze, wobei Natrium-, Kalium-, Ammonium- oder Alkylaminsalze, bevorzugt sind. Bevorzugt sind Trialkylaminsalze, in denen die Alkylgruppen unabhängig voneinander 1 bis 4 Kohlenstoffatome enthalten, insbesondere Triäthylaminsalze.

Gemäss Formel I sind unter Alkyl als selbstständige Gruppe sowie als Teil einer Gruppe von $R_1$ bis $R_5$ gerad- und verzweigtkettige Alkyle zu verstehen. Dazu zählen die Methyl-, die Aethyl- sowie die Isomeren der Propyl- und der Butyl- und der Pentylgruppe. Halogen steht für Fluor, Chlor, Brom oder Jod, insbesondere für Fluor oder Chlor.

Alkoxygruppen sind Aethoxy, n-Propoxy, Isopropoxy und vorzugsweise Methoxy.

Die Verbindungen der Formel I werden hergestellt, indem man reagieren lässt:

a) einen Ester der Formel II

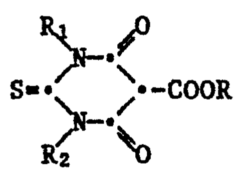

(II)

mit einem Anilinderivat der Formel III

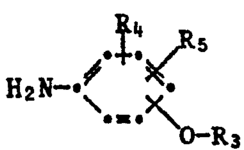

(III)

worin R ein $C_1$-$C_4$-Alkyl oder ein gegebenenfalls durch Nitro substituiertes Phenyl darstellt und die Reste $R_1$ bis $R_5$ die unter der Formel I angegebenen Bedeutungen besitzen, oder

b) eine substituierte Thiobarbitursäure der Formel IV

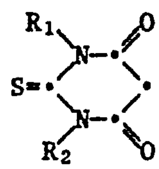

(IV)

mit einem substituierten Phenylisocyanat der Formel (V)

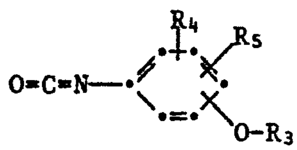

(V)

worin die Reste $R_1$ bis $R_5$ die unter Formel I angegebenen Bedeutungen besitzen,
oder

c) eine substituierte Thiobarbitursäure der Formel IV mit einem substituierten Benzoylazid der Formel VI

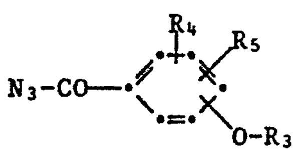

(VI)

worin die Reste $R_1$ bis $R_5$ die unter Formel I angegebene Bedeutungen besitzen.

Die Herstellungsvarianten (a) und (c) werden bei Reaktionstemperaturen von 50° bis 250°C, vorzugweise 70° bis 220°C, durchgeführt. Variante (b) erfordert Reaktionstemperaturen von 0° bis 220°C, insbesondere 0° bis 200°C. Die Reaktionen (a), (b) und (c) finden bei normalem oder erhöhtem Druck ohne oder vorzugsweise in reaktionsinerten oder die Reaktion begünstigenden Lösungs- oder Verdünnungsmitteln statt, wobei in manchen Fällen vorteilhafterweise in Gegenwart einer Base gearbeitet wird.

Die Herstellung der erfindungsgemässen Salze von Verbindungen der Formel I erfolgt durch übliche Neutralisation der freien Säure mit einer Base, insbesondere einer physiologisch verträglichen Base.

Vorzugsweise genannt seien Alkalisalze wie Natrium-, Kalium- oder Lithiumsalze sowie Ammoniumsalze und Trialkylaminsalze wie z.B. das bevorzugte Triethylaminsalz. Die Neutralisation wird in einem reaktionsinerten polaren Lösungsmittel, z.B. einem Alkanol, Ester oder einer etherartigen Verbindung, durchgeführt.

Für die Herstellung der erfindungsgemässen Wirksubstanzen geeignete Lösungs- oder Verdünnungsmittel sind z.B. Ether und etherartige Verbindungen wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Als Basen kommen organische und anorganischen Basen in Betracht; z.B. vorzugsweise tertiäre Amine wie Trialkylamine (z.B. Trimethylamin, Triethylamin oder Tripropylamin), Pyridin und Pyridinbasen (z.B. 4-Dimethylaminopyridin, oder 4-Pyrrolidylaminopyridin), Picoline und Lutidine sowie Oxide, Hydroxide, Carbonate und Hydrogencarbonate von Alkali- und Erdalkalimetallen (z.B. CaO, BaO, NaOH, KOH, $Ca(OH)_2$, $KHCO_3$, $NaHCO_3$, $Ca(HCO_3)_2$, $K_2CO_3$ oder $Na_2CO_3$), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriummethylat. Die Base wird vorteilhafterweise in bezug auf die Reaktanden in 10 bis 100 % der äquimolaren Menge zugesetzt.

In manchen Fällen kann es von Vorteil sein, die Reaktion unter Schutzgasatmosphäre durchzuführen. Geeignete Schutzgase sind z.B. Stickstoff, Helium, Argon oder Kohlendioxid.

Die freie Säure der Formel I führt durch Umsetzung mit Basen zu den ebenfalls zur Erfindung gehörenden Salzen.

Die erfindungsgemässen Wirkstoffe der Formel I können in unterschiedlichen tautomeren Formen vorliegen, nämlich in der Keto- oder Enolform oder in einem Gemisch aus Keto- und Enolform. Die vorliegende Erfindung betrifft sowohl die einzelnen Tautomeren als auch deren Gemische, aber auch die Salze jeder dieser Formen und deren Herstellung.

Das beschriebene Herstellungsverfahren einschliesslich aller Varianten (a), (b) und (c) ist ein Bestandteil vorliegender Erfindung.

Die in den Herstellungsvarianten (a), (b) und (c) genannten Ausgangsstoffe sind teilweise bekannt (siehe z.B. DE-OS 2 936 457) oder können, falls neu, in analoger Weise wie die bekannten Substanzen hergestellt werden.

Neu als Ausgangsverbindungen sind Isocyanate der Formel Va

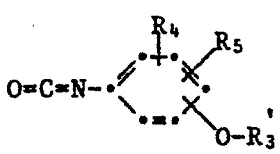

(Va)

worin $R_3$' unsubstituiertes Pyridyl oder ein- bis dreifach durch $C_1$-$C_5$-Alkyl, Halogen, Nitro oder $C_1$-$C_5$-Halogenalkyl mit 1 bis 5 Halogenatomen substituiertes Pyridyl und
$R_4$ und $R_5$ unabhängig voneinander Wasserstoff, Halogen, $C_1$-$C_5$-Alkyl, $C_1$-$C_2$-Halogenalkyl mit 1 bis 3 Halogenatomen, $C_1$-$C_3$-Alkoxy oder Nitro bedeuten.

Die neuen Isocyanate der Formel Va werden nach bekannten Verfahren (vgl. Houben-Weyl, Method. d. Organ. Chemie, Twitchett, H.J., Chem. Soc. Rev. 3, (1974), 209-230) wie folgt hergestellt:

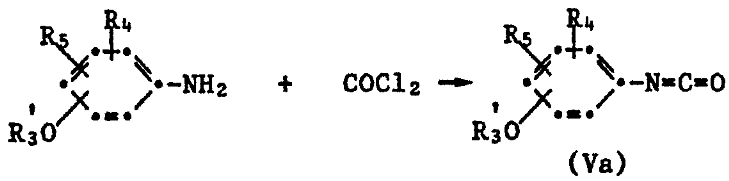


(Va)

worin $R_3'$, $R_4$ und $R_5$ die vorstehend unter Formel Va angegebenen Bedeutungen besitzen. Die Verbindungen der Formeln Va und VIa stellen einen weiteren Gegenstand vorliegender Erfindung dar.

Die Reaktion findet bei Temperaturen von 50° bis 150°C in Gegenwart von reaktionsinerten oder die Reaktion begünstigenden Lösungs- oder Verdünnungsmitteln statt.

Die Azide der Formel VIa werden nach bekannten Verfahren [vgl. Patai, Chemistry of the Acido Group, pp. 503-554 (1971), Interscience Publ. New York] wie folgt hergestellt:

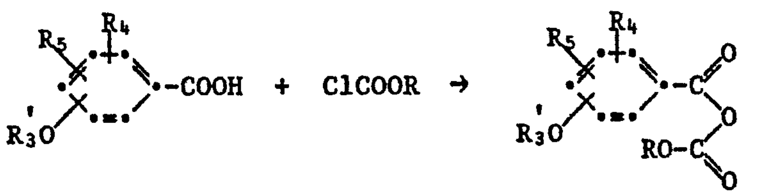


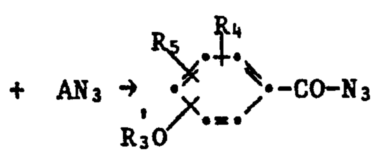


(VIa)

worin $R'_3$, $R_4$ und $R_5$ die vorstehend unter Formel Va angegebenen Bedeutungen besitzen, R eine $C_1$-$C_4$-Alkyl- oder eine gegebenenfalls durch Nitro substituierte Phenylgruppe und A Alkali darstellen.

Die Reaktion findet bei Temperaturen von -50° bis 30°C in Gegenwart von reaktionsinerten oder die Reaktion begünstigenden Lösungs- oder Verdünnungsmitteln statt.

Bei den vorstehend genannten Verfahren zur Herstellung von Verbindungen der Formeln Va und VIa können beispielsweise folgende Lösungs- oder Verdünnungsmittel Verwendung finden: Ether und etherartige Verbindungen wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Neu sind ebenfalls die Ausgangssubstanzen der Formel II

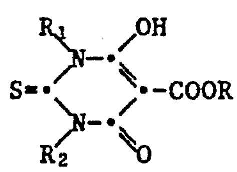

(II)

in welcher R $C_1$-$C_4$-Alkyl oder eine gegebenenfalls durch Nitro substituierte Phenylgruppe darstellt und $R_1$ und $R_2$ die unter Formel I angegebenen Bedeutungen besitzen. Diese Verbindungen sind gleichfalls ein Gegenstand vorliegender Erfindung.

Die Herstellung der Verbindungen der Formel II erfolgt, indem man ein Thiobarbitursäurederivat der Formel IV

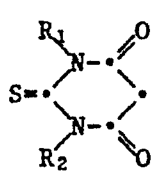

(IV)

mit einem Chlorameisensäureester der Formel

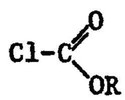

worin $R_1$, $R_2$ und R die oben angegebene Bedeutungen besitzen, in Gegenwart einer Base, z.B. Pyridin, umsetzt.

Die Reaktion wird bei Temperaturen von -50° bis 50°C, vorzugsweise 0° bis 30°C, gegebenenfalls in Gegenwart von reaktionsinerten oder die Reaktion fördernden Lösungs- oder Verdünnungsmitteln durchgeführt, wobei bei deren Abwesenheit die eingesetzte Base als alleiniges Lösungsmittel dient. Als Lösungs- und Verdünnungsmittel können beispielsweise die vorgehend für die Verwendung in den Verfahrensvarianten (a), (b) und (c) beschriebenen eingesetzt werden.

Als besonders vorteilhaft für die Herstellung der erfindungsgemässen Verbindungen der Formel I ist ein Eintopf-Verfahren anzusehen, bei welchem zuerst Verbindungen der Formel II, wie vorstehend beschrieben, aus Verbindungen der Formel IV mit Chlorameisensäureester gebildet und diese anschliessend ohne deren Isolierung mit Verbindungen der Formel III gemäss der vorstehenden Verfahrensvariante (a) wie folgt umgesetzt werden:

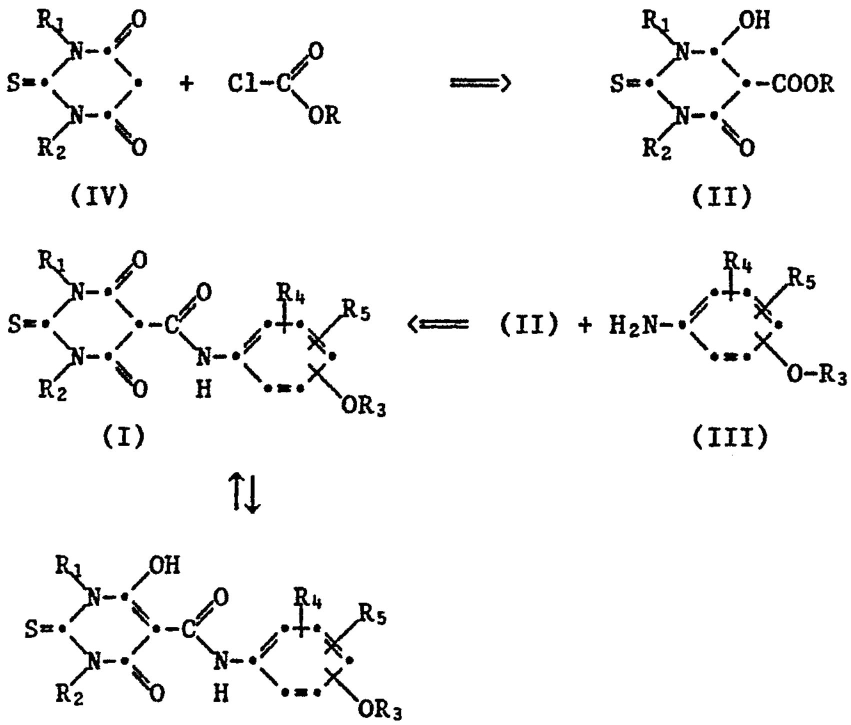

(IV) (II)

(I) (II) + (III)

In den Tabellen 1, 2 und 3 sind Beispiele für Verbindungen der Formeln III, V/Va und VI/VIa dargestellt, welche sich nach den vorstehend beschriebenen Methoden herstellen lassen.

Wie allgemein bekannt, verursachen unter den bei Warmblütern vorkommenden Endoparasiten namentlich die Helminthen bei den von ihnen heimgesuchten Tieren grosse Schäden. Solche durch Helminthiasen verursachten Schäden können bei chronischem und vor allem bei epidemischem Auftreten der Wurmerkrankungen in Viehherden ein volkswirtschaftlich ins Gewicht fallendes Ausmass annehmen. Sie äussern sich bei den erkrankten Tieren unter anderem in Produktivitäts-Einbussen, geschwächter Widerstandskraft gegenüber weiteren Krankheiten und erhöhter Mortalität. Besonders gefährliche Wurmkrankheiten werden durch im Magen-Darmtrakt und anderen Organen parasitierende Helminthen hervorgerufen und können beispielsweise bei Wiederkäuern wie Rindern, Schafen und Ziegen sowie Pferden, Schweinen, Geflügel, Rotwild, Hunden und Katzen auftreten.

In der vorliegenden Beschreibung werden unter dem Begriff Helminthen insbesondere parasitische Würmer verstanden, die zu den Phyla Plathelminthes (Cestoden, Trematoden) und Nemathelminthes (Nematoden und Verwandte) gehören, also Bandwürmer, Saugwürmer und Rundwürmer des Gastrointestinal-Traktes und anderer Organe (z.B. Leber, Lunge, Niere, Lymphgefässe, Blut etc.).

Es ist deshalb eine vordringliche Aufgabe, therapeutische Mittel zu entwickeln, die sich zur Bekämpfung von Helminthen in allen ihren Entwicklungsstadien sowie zur Vorbeugung gegen den Befall durch diese Parasiten eignen.

Es sind zwar eine Reihe von Stoffen mit anthelmintischer Wirkung bekannt, die für die Bekämpfung der verschiedenen Helminthen-Species vorgeschlagen wurden. Diese vermögen jedoch nicht voll zu befriedigen, sei es, dass bei verträglicher Dosierung eine Ausschöpfung ihres Wirkungsspektrums nicht möglich ist, oder dass sie in therapeutisch wirksamen Dosen unerwünschte Nebenwirkungen oder Eigenschaften zeigen. In diesem Zusammenhang spielt auch die heute vermehrt auftretende Resistenz gegen bestimmte Stoffklassen eine immer bedeutendere Rolle. Das beispielsweise in der Literatur beschriebene "Albendazol" (British Pat. No. 1464326; Am. J. Vet. Res. 38, 1425-1426 (1977); Am. J. Vet. Res. 37, 1515-1516 (1976); Am. J. Vet. Res. 38, 807-808 (1977); Am. J. Vet. Res. 38, 1247-1248 (1977)) besitzt nur ein begrenztes anthelmintisches Wirkungsspektrum bei Wiederkäuern. Seine Wirkung gegen Benzimidazol-resistente Nematoden und adulte Leberegel ist völlig unzureichend, wobei vor allem die pathogen wichtigen unreifen Wanderformen der letzteren bei den für das Wirtstier verträglichen Dosierungen nicht angegriffen werden.

Ferner sind in der Deutschen Offenlegungsschrift 2 405 732 Amidocarbonylthiobarbitursäurederivate als Ekto- und Endoparasitizide beschrieben.

Die erfindungsgemässen neuen Wirkstoffe der Formel I unterscheiden sich strukturell in charakteristischer Weise von den aus der obengenannten Publikation bekannten Barbitursäurederivaten. Dazu wurde überraschenderweise gefunden, dass die neuen Verbindungen ein breites Wirkungsspektrum gegen im Tierorganismus, vor allem in Säugetieren, parasitierende Helminthen wie Nematoden, Cestoden und Trematoden besitzen, wobei ihre Wirkung sich vorzugsweise gegen Nematoden (Rundwürmer) richtet.

Als besonderes Merkmal der Verbindungen der Formel I ist ihre gegenüber Warmblütern überraschend hohe Verträglichkeit hervorzuheben, die sie gegenüber den bekannten Thiobarbitursäurederivaten überlegen macht. Ihre praktische Handhabung bei der Behandlung Wurm-erkrankter Tiere wird dadurch ausserordentlich erleichtert, da sie auch in höheren Dosen von den medikierten Tieren symptomlos vertragen werden.

Die erfindungsgemässen neuen Wirkstoffe der Formel I eignen sich beispielsweise zur Bekämpfung parasitärer Nematoden der Ordnungen (nach K.I. Skrajabin)

Rhabditida
Ascaridida
Spirurida
Trichocephalida

oder zur Bekämpfung von Cestoden der Ordnungen (nach Wardle & McLeod)

Cyclophyllidae
Pseudophyllidae

oder zur Bekämpfung von Trematoden der Ordnung

Digenea

bei Haus- und Nutztieren wie Rindern, Schafen, Ziegen, Pferden, Schweinen, Rotwild, Katzen, Hunden und Geflügel. Sie können den Tieren sowohl als Einzeldosis als auch wiederholt verabreicht werden, wobei die einzelnen Gaben je nach Tierart vorzugsweise

zwischen 1 und 20 mg pro kg Körpergewicht betragen. Durch eine protrahierte Verabreichung kann in manchen Fällen eine bessere Wirkung erzielt oder mit geringeren Gesamtdosen ausgekommen werden.

Die erfindungsgemäsen Mittel werden hergestellt, indem die Wirkstoffe der Formel I mit flüssigen und/oder festen Formulierungshilfsstoffen durch schrittweises Vermischen und/oder Vermahlen derart in Kontakt gebracht werden, dass eine anwendungskonforme optimale Entfaltung der anthelmintischen Aktivität der Formulierung erzielt wird.

Die Formulierungsschritte können durch Kneten, Granulieren (Granulate) und gegebenenfalls Pressen (Pellets) ergänzt werden.

Als Formulierungshilfsstoffe dienen beispielsweise feste Trägerstoffe, Lösungsmittel und gegebenenfalls oberflächenaktive Stoffe (Tenside).

Zur Bereitung der erfindungsgemässen Mittel werden folgende Formulierungshilfsstoffe verwendet:
Feste Trägerstoffe wie z.B. Kaolin, Talkum, Bentonit, Kochsalz, Calciumphosphat, Kohlenhydrate, Cellulosepulver, Baumwollsaatmehl, Polyäthylenglykoläther, gegebenenfalls Bindemittel wie z.B. Gelatine, lösliche Cellulosederivate, gewünschtenfalls unter Zusatz von oberflächenaktiven Stoffen wie ionischen oder nicht-ionischen Dispersionsmitteln; ferner natürliche Gesteinsmehle wie Calcit, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden.

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat; aliphatische Kohlenwasserstoffe wie z.B. Cyclohexan oder Paraffine, Alkohole und Glykole sowie deren Ether und Ester, wie z.B. Ethanol, Ethylenglykol, Ethylenglykolmonomethyl- oder -ethylether, Ketone wie z.B. Cyclohexanon, stark polare Lösungsmittel wie z.B. N-Methyl-2-pyrrolidon, Dimethylsulfoxid oder Dimethylformamid, sowie gegebenenfalls epoxydierte Pflanzenöle wie z.B. epoxydiertes Kokosnussöl oder Sojaöl und Wasser.

Als oberflächenaktive Verbindungen kommen je nach Art des zu formulierenden Wirkstoffes der Formel I nicht-ionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyltaurinsalze zu erwähnen.

Häufig werden sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz

der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes oder Phospholipide in Frage.

Als nicht-ionische Tenside kommen in erster Linie Polyglykoletherderivate von aliphatischen oder cycloaliphatischen Alkoholen, gesättigten oder ungesättigten Fettsäuren und Alkyphenolen in Frage, die 3 bis 30 Glykolethergruppen und 8 bis 20 Kohlenstoffatome im aliphatischen Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Ethylenglykoleinheiten.

Als Beispiele nicht-ionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:
"Mc Cutcheon's Detergents and Emulsifiers Annual",
MC Publishing Corp., Ridgewood New Jersey, 1980;
Sisley and Wood, "Encyclopedia of Surface Active Agents",
Chemical Publishing Co., Inc. New York, 1980.

Als Bindemittel für Tabletten und Boli kommen chemisch abgewandelte, in Wasser oder Alkohol lösliche, polymere Naturstoffe in Frage, wie Stärke-, Cellulose- oder Protein-derivate (z.B. Methylcellulose, Carboxymethylcellulose, Ethylhydroxyethylcellulose, Proteine wie Zein, Gelatine und dergleichen) sowie synthetische Polymere wie z.B. Polyvinylalkohol, Polyvinylpyrrolidon etc.. Ferner sind in Tabletten Füllstoffe, (z.B. Stärke, mikrokristalline Cellulose, Zucker, Milchzucker etc.), Gleitmittel und Sprengmittel enthalten.

Liegen die anthelmintischen Mittel in Form von Futterkonzentraten vor, so dienen als Trägerstoffe z.B. Leistungsfutter, Futtergetreide oder Proteinkonzentrate. Solche Futterkonzentrate oder -mittel können ausser den Wirkstoffen noch Zusatzstoffe, Vitamine, Antibiotika, Chemotherapeutika, oder andere Pestizide, vornehmlich Bakteriostatika, Fungistatika, Coccidiostatika, oder auch Hormonpräparate, Stoffe mit anaboler Wirkung oder das Wachstum begünstigende, die Fleischqualität von Schlachttieren beeinflussende oder in anderer Weise für den Organismus nützliche Stoffe enthalten. Werden die Mittel oder die darin enthaltenen Wirkstoffe der Formel I direkt

dem Futter oder den Viehtränken zugesetzt, so enthält das Fertigfutter oder die Fertigtränke die Wirkstoffe vorzugsweise in einer Konzentration von etwa 0,0005 bis 0,02 Gewichtsprozent (5-200 ppm).

Die Applikation der erfindungsgemässen Mittel an die zu behandelnden Tiere kann peroral, parenteral oder subcutan durchgeführt werden, wobei die Mittel in Form von Lösungen, Emulsionen, Suspensionen (Drenchs), Pulvern, Tabletten, Boli und Kapseln vorliegen.

Die erfindungsgemässen anthelmintischen Mittel enthalten in der Regel 0,1 bis 99 Gew.-%, insbesondere 0,1 bis 95 Gew.-% Wirkstoff der Formel I, 99,9 bis 1 Gew.-%, insbesondere 99,8 bis 5 Gew.-%, eines festen oder flüssigen Zusatzstoffes, darunter 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Derartige vom Endverbraucher verwendete anthelmintische Mittel sind ebenfalls ein Bestandteil der vorliegenden Erfindung.

In jedem der erfindungsgemässen Verfahren zur Schädlingsbekämpfung bzw. der erfindungsgemässen Schädlingsbekämpfungsmittel können die Wirkstoffe der Formel I in allen tautomeren Formen, deren Mischungen oder in Form ihrer Salze eingesetzt werden.

Die Erfindung schliesst auch ein Verfahren zum prophylaktischen Schutz von Tieren gegen parasitäre Helminthen ein, das dadurch gekennzeichnet ist, dass man die Wirkstoffe der Formel I bzw. die Wirkstofformulierungen als Zusatz zum Futter oder zu den Tränken oder auch in fester oder flüssiger Form oral, durch Injektion oder parenteral den Tieren appliziert.

Die nachfolgenden Beispiele dienen der Illustration der Erfindung, ohne sie einzuschränken.

## 1. Herstellungsbeispiele

### 1.1. 4-(5-Trifluormethylpyridyl-2-oxy)-phenylisocyanat

20,3 g (0,08 Mol) 4-(5-Trifluormethylpyridyl-2-oxy)-anilin werden in 200 ml trockenem Chlorbenzol gelöst, auf -20°C gekühlt und rasch eine Lösung von 3,7 g HCl-Gas in 80 ml trockenem Chlorbenzol zugegeben. Der entstandene Kristallbrei wird auf -30°C gekühlt und in kräftigem Strom ca. 40 g Phosgen innerhalb einer Stunde eingeleitet. Jetzt wird die Phosgenzufuhr gedrosselt und noch eine Stunde bei 20°C, danach im Oelbad eine Stunde bei 110°C gerührt. Zuletzt entsteht eine klare Lösung. Man lässt nun im Stickstoffstrom abkühlen und treibt unter Rühren mit Stickstoff den Phosgenüberschuss aus. Danach wird zuerst unter Normaldruck das Chlorbenzol, danach im Hochvakuum das Isocyanat abdestilliert.
Sdp. $_{0,03\ mbar}$ 105-107°C; Ausbeute > 90 %.

### 1.2. 1,3-Dimethyl-5-[4-chlor-2-(2,6-dichlorphenoxy)-phenylcarbamoyl]-2-thiobarbitursäure

a) 1,3-Dimethyl-5-äthoxycarbonyl-2-thiobarbitursäure

155 g (0,9 Mol) 1,3-Dimethyl-2-thio-barbitursäure, 88,3 g (1,12 Mol) Pyridin und 9 g 4-Dimethylaminopyridin werden in 660 ml Dichlormethan gelöst und auf 0°C abgekühlt. Danach werden 102 g (0,94 Mol) Chlorameisensäureäthylester innerhalb einer Stunde zugetropft. Die Mischung wird anschliessend 12 Stunden bei 0°C gerührt. Danach lässt man die Mischung auf Raumtemperatur erwärmen und arbeitet sie nach weiteren 7 Stunden folgendermassen auf. Es werden 600 ml Dichlormethan zugegeben und die Lösung dreimal mit je 1000 ml Wasser gewaschen, über $Na_2SO_4$ getrocknet und das Lösungsmittel abgedampft. Das Rohprodukt wird aus Ethanol umkristallisiert. Smp. 90-93°C.

b) 2,5 g (10 mMol) 1,3-Dimethyl-5-äthoxycarbonyl-2-thiobarbitursäure, 2,9 g (10 mMol) 4-Chlor-2-(2,6-dichlorphenoxy)-anilin, 40 ml Aethanol und 3 ml Dimethylformamid werden gemischt und unter Rühren 6 Stunden am Rückfluss gekocht. Dann lässt man abkühlen und filtriert das gebildete Kristallisat ab. Es kann nötigenfalls aus einer Mischung von Dioxan und Aethanol umkristallisiert werden. Smp. 254-257°C.

1.3. <u>1,3-Dimethyl-5-[4-(5-trifluormethyl-pyridyl-2-oxy)-phenylcarbamoyl]-2-thiobarbitursäure</u>

2,5 g (10 mM) 1,3-Dimethyl-5-äthoxycarbonyl-2-thiobarbitursäure, 2,55 g (10 mM) 4-(5-Trifluormethyl-pyridyl-2-oxy-)anilin und 5 ml Dimethylformamid werden 6 Stunden bei 118°C gerührt. Danach lässt man abkühlen, verreibt mit Wasser und filtriert das Kristallisat ab. Das Rohprodukt wird aus Aethanol umkristallisiert. Smp. 156-158°C.

1.4. <u>1-Methyl-3-äthyl-5-[4-chlor-2-(4-chlorphenoxy)-phenylcarbamoyl]-2-thiobarbitursäure</u>

3,4 g (20 mMol) 1-Methyl-3-äthyl-2-thiobarbitursäure werden in 80 ml Xylol suspendiert. Man gibt 1 g (8 mMol) Triäthylamin hinzu, erwärmt auf 40°C und tropft unter Rühren bei 40°C 5,6 g (20 mMol) 4-Chlor-2-(4-chlorphenoxy)-phenylisocyanat, gelöst in 30 ml Xylol, dazu. Man rührt noch 2 Stunden bei 40°C und lässt dann abkühlen. Dann gibt man 50 ml 2N HCl hinzu, filtriert das ausgefallene Kristallisat ab und wäscht mit Wasser. Nach dem Trocknen bei 80°C erhält man ein Produkt vom Smp. 250-251°C.

1.5. <u>1,3-Dimethyl-5-[4-chlor-2-(2,6-dichlor-phenoxy)-phenylcarbamoyl]-2-thiobarbitursäure</u>

15,5 g (0,09 Mol) 1,3-Dimethyl-2-thiobarbitursäure, 8,8 g (1,1 Mol) Pyridin und 0,9 g 4-Dimethylaminopyridin werden in 65 ml Dichlormethan gelöst und auf 0°C abgekühlt. Danach werden 10,2 g (0,094 Mol) Chlorameisensäureäthylester innerhalb einer Stunde zugetropft. Die Mischung wird noch 12 Stunden bei 0°C, danach 7 Stunden bei Raumtemperatur gerührt. Das Lösungsmittel wird weitgehend abgedampft, 26,1 g (0,09 Mol) 4-Chlor-2-(2,6-dichlorphenoxy)-anilin,

gelöst in 360 ml Aethanol und 30 ml Dimethylformamid, hinzugegeben und unter Rühren 6 Stunden am Rückfluss gekocht. Danach lässt man abkühlen, filtriert das entstandene Kristallisat ab und kristallisiert nötigenfalls aus Dioxan/Aethanol um. Smp. 254-257°C.

1.6. 1,3-Dimethyl-5-[4-(2-chlor-4-trifluormethyl-phenoxy)-2,6-dimethylphenylcarbamoyl]-2-thiobarbitursäure

a) 6,4 g (0,02 Mol) 2,6-Dimethyl-4-(2-chlor-4-trifluormethyl-phenoxy)-benzoesäure werden in 50 ml Aceton bei 0°C mit 2,7 g (0,025 Mol) Chlorameisensäureäthylester und danach innerhalb von 15 Min. mit 2,2 g (0,022 Mol) Triäthylamin versetzt. Danach werden, ebenfalls bei 0°C, 2,0 g (0,03 Mol) Natriumazid, gelöst in 7 ml Wasser, zugegeben. Man rührt noch 3 Stunden bei 0°C und schüttet das Gemisch dann auf 100 ml Eiswasser. Es wird mit Toluol extrahiert und die Toluolphase mit $Na_2SO_4$ getrocknet. Die Toluolphase enthält das Produkt 2,6-Dimethyl-4-(2-chlor-4-trifluormethylphenoxy)-benzoylazid.

b) 3,44 g (0,02 Mol) 1,3-Dimethyl-2-thiobarbitursäure und 2,0 g (0,02 Mol) Triäthylamin werden in 40 ml Toluol gelöst und auf 90°C erwärmt. Bei dieser Temperatur wird die gesamte in der Stufe a) erhaltene Toluollösung des 2,6-Dimethyl-4-(2-chlor-4-trifluormethylphenoxy)-benzoylazids zugetropft. Die Zutropfgeschwindigkeit richtet sich nach der Stärke der Stickstoffentwicklung. Nach beendetem Zutropfen erhöht man die Temperatur bis zum Rückfluss und kocht, bis kein Stickstoff mehr entweicht. Man lässt dann abkühlen, filtriert den Niederschlag ab und wäscht mit wenig Aethanol nach. Das so gebildete Thiobarbitursäuretriäthylammoniumsalz wird in 1N HCl suspendiert, abfiltriert und danach gründlich mit Wasser gewaschen. Man erhält das Endprodukt. Smp. 171-173°C.

1.7. 1,3-Dimethyl-5-[3-(2-chlor-4-trifluormethyl-phenoxy)-4-methoxyphenylcarbamoyl]-2-thiobarbitursäure

a) In einem Sulfierkolben mit Wasserabscheider wird ein Gemisch von 33,6 g (0,2 Mol) 3-Hydroxy-4-methoxybenzoesäure und 26,8 g (0,4 Mol) Kaliumhydroxid (85 %) in 200 ml Toluol und 200 ml Dimethylsulfoxid

bei 140°C entwässert. Nach Abdestillieren des Toluols wird das Gemisch auf 90°C abgekühlt und mit 43 g (0,2 Mol) 3,4-Dichlorbenzotrifluorid, 1,1 g Kaliumjodid und 0,2 g Kupferspäne in 20 ml Dimethylsulfoxid versetzt. Das Gemisch wird 30 Stunden lang auf 150°C gehalten und dann das Lösungsmittel im Hochvakuum abdestilliert. Der Rückstand wird in 250 ml Wasser gelöst, abfiltriert, mit 100 ml 2N HCl in 200 g Eis angesäuert, abgenutscht und mit Wasser gewaschen. Nach Trocknen unter Vakuum bei 80°C erhält man 58 g 3-(2-Chlor-4-trifluormethyl-phenoxy)-4-methoxybenzoesäure.
Smp. 162-165°C.

b) 6,4 g (0,02 Mol) 3-(2-Chlor-4-trifluormethyl-phenoxy)-4-methoxybenzoesäure werden in 50 ml Aceton bei 0°C mit 2,7 g (0,025 Mol) Chlorameisensäureäthylester und danach innerhalb von 15 Min. mit 2,2 g (0,022 Mol) Triäthylamin versetzt. Danach werden, ebenfalls bei 0°C, 2,0 g (0,03 Mol) Natriumazid, gelöst in 7 ml Wasser, zugegeben. Man rührt noch 3 Stunden bei 0°C und schüttet das Gemisch dann auf 100 ml Eiswasser. Es wird mit Toluol extrahiert und die Toluolphase mit $Na_2SO_4$ getrocknet. Die Toluolphase enthält das Produkt 3-(2-Chlor-4-trifluormethyl-phenoxy)-4-methoxybenzoylazid.

c) 3,44 g (0,02 Mol) 1,3-Dimethyl-2-thiobarbitursäure und 2,0 g (0,02 Mol) Triäthylamin werden in 40 ml Toluol gelöst und auf 90°C erwärmt. Bei dieser Temperatur wird die gesamte in der Stufe b) erhaltene Toluollösung des 3-(2-Chlor-4-trifluormethyl-phenoxy)-4-methoxybenzoylazids zugetropft. Die Zutropfgeschwindigkeit richtet sich nach der Stärke der Stickstoffentwicklung. Nach beendetem Zutropfen erhöht man die Temperatur bis zum Rückfluss und kocht, bis kein Stickstoff mehr entweicht. Man lässt dann abkühlen, filtriert den Niederschlag ab und wäscht mit wenig Aethanol nach. Das so gebildete Thiobarbitursäuretriäthylammoniumsalz wird in 1N HCl suspendiert, abfiltriert und danach gründlich mit Wasser gewaschen. Man erhält das Endprodukt. Smp. 165-168°C.

1.8. 1,3-Dimethyl-5-[3-(3,5-dichlor-pyridyl-2-oxy)-4-methoxyphenylcarbamoyl]-2-thiobarbitursäure

a) In einem Sulfierkolben mit Wasserabscheider wird ein Gemisch von 14,0 g (0,1 Mol) 5-Amino-2-methoxy-phenol und 6,7 g (0,1 Mol) Kaliumhydroxid (85 %) in 100 ml Toluol und 100 ml Dimethylsulfoxid bei 150°C entwässert. Nach 3 Stunden wird das Toluol abdestilliert, anschliessend das Gemisch auf 80°C abgekühlt und mit 18,2 g (0,1 Mol) 2,3,5-Trichlorpyridin in 25 ml Dimethylsulfoxid versetzt. Das Gemisch wird 3 Stunden bei 120°C gerührt, abgekühlt, mit 5 ml Essigsäure versetzt und im Hochvakuum abdestilliert. Der Rückstand wird mit 200 ml Wasser und 300 ml Methylenchlorid extrahiert. Die organische Phase liefert 28 g 3-(3,5-Dichlor-pyridyl-2-oxy)-4-methoxy-anilin als erstarrendes Oel. Smp. 95-105°C.

b) 3,7 g (15 mM) 1,3-Dimethyl-5-äthoxycarbonyl-2-thiobarbitursäure, 4,3 g (15 mM) 3-(3,5-dichlor-pyridyl-2-oxy-)-4-methoxy-anilin und 13 ml Dimethylformamid werden 20 Stunden bei 60°C gerührt. Danach lässt man abkühlen, verreibt mit Wasser und filtriert das Kristallisat ab. Das Rohprodukt wird in Aethanol suspendiert und abgenutscht. Smp. 179-180°C.

1.9. 1,3-Dimethyl-5-[4-(2-chlor-4-trifluormethyl-phenoxy)-3-methoxyphenylcarbamoyl]-2-thiobarbitursäure

3,7 g (15 mM) 1,3-Dimethyl-5-äthoxycarbonyl-2-thiobarbitursäure, 4,8 g (15 mM) 4-(2-Chlor-4-trifluormethyl-phenoxy)-3-methoxy-anilin und 13 ml Dimethylformamid werden 20 Stunden bei 60°C gerührt. Danach lässt man abkühlen, verreibt mit Wasser und filtriert das Kristallisat ab. Das Rohprodukt wird in Aethanol suspendiert und abgenutscht. Smp. 178-182°C.

Tabelle 1: Verbindungen der Formel

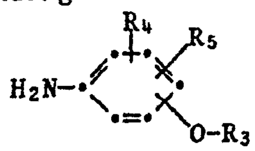

| Verb. Nr. | $R_4$ | $R_5$ | $-OR_3$ | physikal. Konst. [°C] [mbar] |
|---|---|---|---|---|
| 3.1 | H | H | $4-O-C_6H_4-CF_3(3)$ | ölig |
| 3.2 | $2-CH_3$ | $6-CH_3$ | $4-O-C_6H_4-CF_3(4)$ | Sdp.126/0.01 |
| 3.3 | $2-CH_3$ | $6-CH_3$ | $4-O-C_6H_3-Cl(3)-CF_3(4)$ | Sdp.135/0.01 |
| 3.4 | $2-CH_3$ | $6-CH_3$ | $4-O-C_6H_3-Cl(2)-CF_3(4)$ | Smp.104-106 |
| 3.5 | $2-C_3H_7$iso | H | $4-O-C_6H_4-CF_3(4)$ | Sdp.127/0.01 |
| 3.6 | $2-C_3H_7$iso | H | $4-O-C_6H_4-CF_3(3)$ | Sdp.140/0.02 |
| 3.7 | $2-C_3H_7$iso | H | $4-O-C_6H_3-Cl(3)-CF_3(4)$ | Sdp.145-150/ 0.01 |
| 3.8 | H | H | $4-O-C_6H_4-CF_3(4)$ | Smp. 77-79 |
| 3.9 | $2-CH_3$ | H | $4-O-C_6H_4-CF_3(4)$ | Smp.120/0.01 |
| 3.10 | H | H | $4-O-Pyridyl(2)-CF_3(5)$ | ölig |
| 3.11 | H | H | $4-O-Pyridyl(2)-Cl(3)-CF_3(5)$ | Sdp.133-135/ 0.03 |
| 3.12 | $2-C_3H_7$iso | H | $4-O-Pyridyl(2)-CF_3(5)$ | Sdp.121-126/ 0.02 |
| 3.13 | H | H | $4-O-Pyridyl(2)-NO_2(3)$ | Smp.110-112 |
| 3.14 | H | H | $4-O-Pyridyl(2)-NO_2(5)$ | ölig |
| 3.15 | H | H | $4-O-C_6H_3-CF_3(3)-NO_2(4)$ | ölig |
| 3.16 | H | H | $4-O-C_6H_3-CF_3(2)-NO_2(4)$ | ölig |
| 3.17 | H | H | $4-O-C_6H_3-CF_3(4)-NO_2(2)$ | ölig |
| 3.18 | $2-CH_3$ | $6-CH_3$ | $4-O-C_6H_3-CF_3(4)-NO_2(2)$ | ölig |
| 3.19 | $2-CH_3$ | $6-CH_3$ | $4-O-Pyridyl(2)-CF_3(5)$ | Sdp.125-127/ 0.04 |

| Verb. Nr. | $R_4$ | $R_5$ | $-OR_3$ | physikal. Konst. [°C] [mbar] |
|---|---|---|---|---|
| 3.20 | H | H | 4-O-Pyridyl(2)-$Cl_2$(3,5) | Smp. 72-74 |
| 3.21 | 4-$OCH_3$ | H | 3-O-Pyridyl(2)-$Cl_2$(3,5) | Smp.100-105 |
| 3.22 | 3-$OCH_3$ | H | 4-O-$C_6H_3$-Cl(2)-$CF_3$(4) | ölig |
| 3.23 | H | H | 4-O-Pyridyl(2)-Cl(5)-F(3) | |
| 3.24 | 3-$OCH_3$ | H | 4-O-Pyridyl(2)-Cl(5)-F(3) | |
| 3.25 | 4-$OCH_3$ | H | 3-O-Pyridyl(2)-Cl(5)-F(3) | |

Tabelle 2: Verbindungen der Formel

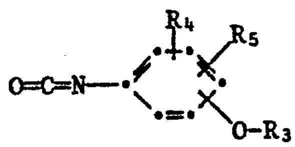

| Verb. Nr. | $R_4$ | $R_5$ | $-OR_3$ | physikal. Konst. [°C] [mbar] |
|---|---|---|---|---|
| 5.1 | H | H | 4-O-$C_6H_4$-$CF_3$(3) | ölig |
| 5.2 | 2-$CH_3$ | 6-$CH_3$ | 4-O-$C_6H_4$-$CF_3$(4) | Sdp.118/0.03 |
| 5.3 | 2-$C_3H_7$iso | H | 4-O-$C_6H_4$-$CF_3$(4) | Sdp.123/0.2 |
| 5.4 | 2-$C_3H_7$iso | H | 4-O-$C_6H_4$-$CF_3$(3) | Sdp.107-110/ 0.05 |
| 5.5 | H | H | 4-O-$C_6H_4$-$CF_3$(4) | Sdp.118-120/ 0.08 |
| 5.6 | H | H | 4-O-Pyridyl(2)-Cl(3)-$CF_3$(5) | ölig |
| 5.7 | H | H | 4-O-Pyridyl(2)-$Cl_2$(3,5) | Smp. 65-66 |

Tabelle 3: Verbindungen der Formel

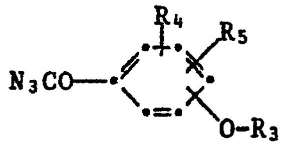

| Verb. Nr. | $R_4$ | $R_5$ | $-OR_3$ | physikal. Konst. [°C] |
|---|---|---|---|---|
| 6.1 | $4-OCH_3$ | H | $3-O-Pyridyl(2)-Cl_2(3.5)$ | |
| 6.2 | $4-OCH_3$ | H | $3-O-C_6H_3-Cl(2)-CF_3(4)$ | |
| 6.3 | $3-OCH_3$ | H | $4-O-C_6H_3-Cl(2)-CF_3(4)$ | |

Als Beispiele für Benzoesäure-Derivate der Formel

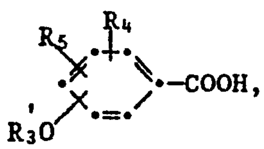

welche sich zur Herstellung von Verbindungen der Formel VI eignen, seien 3-(3,5-Dichlor-pyridyl-2-oxy)-4-methoxy-benzoesäure (Smp. 217-218°C) und 3-(2-Chlor-4-trifluormethyl-phenoxy)-4-methoxy-benzoesäure (Smp. 162-165°C; Beispiel 1.7a) und 4-(2-Chlor-4-trifluormethyl-phenoxy)-3-methoxy-benzoesäure (Smp. 174-176°C) genannt.

Tabelle 4: Verbindungen der Formel

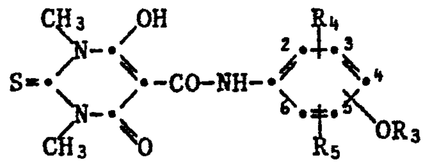

| Verb. Nr. | $R_1$ | $R_2$ | $R_4$ | $R_5$ | $-OR_3$ | physikal. Konst. [°C] |
|---|---|---|---|---|---|---|
| 1.1 | $CH_3$ | $CH_3$ | 4-Cl | H | 2-O-$C_6H_3$-$Cl_2$(2,6) | Smp.254-257 |
| 1.2 | $CH_3$ | $CH_3$ | 4-Cl | H | 2-O-$C_6H_4$-F(4) | Smp.244-247 |
| 1.3 | $CH_3$ | $CH_3$ | H | H | 4-O-$C_6H_4$-($CH_2$CN)(3) | Smp.167-170 |
| 1.4 | $CH_3$ | $CH_3$ | 3-Cl | 5-Cl | 4-O-Pyridyl(2)-Cl(3)-$CF_3$(5) | Smp.231-233 |
| 1.5 | $CH_3$ | $CH_3$ | H | H | 4-O-$C_6H_4$-$CF_3$(3) | Smp.148-149 |
| 1.6 | $CH_3$ | $CH_3$ | 2-$CH_3$ | 6-$CH_3$ | 4-O-$C_6H_4$-$CF_3$(4) | Smp.183-184 |
| 1.7 | $CH_3$ | $CH_3$ | 2-$CH_3$ | 6-$CH_3$ | 4-O-$C_6H_3$-Cl(2)-$CF_3$(4) | Smp.171-173 |
| 1.8 | $CH_3$ | $CH_3$ | 2-$C_3H_7$iso | H | 4-O-$C_6H_4$-$CF_3$(4) | Smp.162-163 |
| 1.9 | $CH_3$ | $CH_3$ | 2-$C_3H_7$iso | H | 4-O-$C_6H_4$-$CF_3$(3) | Smp.111-113 |
| 1.10 | $CH_3$ | $CH_3$ | 2-$C_3H_7$iso | H | 4-O-$C_6H_3$-Cl(3)-$CF_3$(4) | Smp.130-136 |
| 1.11 | $CH_3$ | $CH_3$ | H | H | 4-O-$C_6H_4$-$CF_3$(4) | Smp.165-170 |
| 1.12 | $CH_3$ | $CH_3$ | 2-$CH_3$ | H | 4-O-$C_6H_4$-$CF_3$(4) | Smp.168-176 |
| 1.13 | $CH_3$ | $CH_3$ | H | H | 4-O-Pyridyl(2)-$CF_3$(5) | Smp.156-158 |
| 1.14 | $CH_3$ | $CH_3$ | H | H | 4-O-Pyridyl(2)-$CF_3$(5)• $N(C_2H_5)_3$ | Smp.102-105 |
| 1.15 | $CH_3$ | $CH_3$ | H | H | 4-O-Pyridyl(2)-Cl(3)-$CF_3$(5) | Smp.153-154 |
| 1.16 | $CH_3$ | $CH_3$ | 2-$C_3H_7$iso | H | 4-O-Pyridyl(2)-$CF_3$(5) | Smp.189-190 |
| 1.17 | $CH_3$ | $CH_3$ | H | H | 4-O-Pyridyl(2)-$NO_2$(3) | Smp.210-213 |
| 1.18 | $CH_3$ | $CH_3$ | H | H | 4-O-Pyridyl(2)-$NO_2$(5) | Smp.198-200 |
| 1.19 | $CH_3$ | $CH_3$ | H | H | 4-O-$C_6H_3$-$CF_3$(3)-$NO_2$(4) | Smp.187-189 |
| 1.20 | $CH_3$ | $CH_3$ | H | H | 4-O-$C_6H_3$-$CF_3$(2)-$NO_2$(4) | Smp.193-195 |

| Verb. Nr. | $R_1$ | $R_2$ | $R_4$ | $R_5$ | $-OR_3$ | physikal. Konst. [°C] |
|---|---|---|---|---|---|---|
| 1.21 | $CH_3$ | $CH_3$ | H | H | 4-O-$C_6H_3$-$CF_3$(4)-$NO_2$(2) | Smp.173-174 |
| 1.22 | $CH_3$ | $CH_3$ | 2-$CH_3$ | 6-$CH_3$ | 4-O-$C_6H_3$-$CF_3$(4)-$NO_2$(2) | Smp.172-174 |
| 1.23 | $CH_3$ | $CH_3$ | 2-$CH_3$ | 6-$CH_3$ | 4-O-Pyridyl(2)-$CF_3$(5) | Smp.210-211 |
| 1.24 | $CH_3$ | $CH_3$ | H | H | 4-O-Pyridyl(2)-$Cl_2$-(3,5) | Smp.177-178 |
| 1.25 | $CH_3$ | $CH_3$ | H | H | 4-O-Pyridyl(2)-Cl-(5)-F(3) | Smp.185-187 |
| 1.26 | $CH_3$ | $CH_3$ | H | H | 4-O-Pyridyl(2)-$F_2$-(3,5) | |
| 1.27 | $CH_3$ | $CH_3$ | 4-$OCH_3$ | H | 3-O-Pyridyl(2)-$Cl_2$ (3,5) | Smp.179-180 |
| 1.28 | $CH_3$ | $CH_3$ | 3-$OCH_3$ | H | 4-O-Pyridyl(2)-$Cl_2$ (3,5) | Smp.179-180 |
| 1.29 | $CH_3$ | $CH_3$ | 4-$OCH_3$ | H | 3-O-$C_6H_3$-Cl(2)-$CF_3$(4) | Smp.165-168 |
| 1.30 | $CH_3$ | $CH_3$ | 3-$OCH_3$ | H | 4-O-$C_6H_3$-Cl(2)-$CF_3$(4) | Smp.178-182 |
| 1.31 | $CH_3$ | $CH_3$ | 3-$OCH_3$ | H | 4-O-Pyridyl(2)-Cl(3)-$CF_3$(5) | Smp.205-207 |
| 1.32 | $CH_3$ | $CH_3$ | 4-$OCH_3$ | H | 3-O-Pyridyl(2)-Cl(3)-$CF_3$(5) | Smp.195-197 |
| 1.33 | $CH_3$ | $CH_3$ | 3-$OCH_3$ | H | 4-O-Pyridyl(2)-Cl(5)-F(3) | Smp.170-172 |
| 1.34 | $CH_3$ | $CH_3$ | 4-$OCH_3$ | H | 3-O-Pyridyl(2)-Cl(5)-F(3) | Smp.186-189 |
| 1.35 | $CH_3$ | $CH_3$ | 3-$OCH_3$ | H | 4-O-Pyridyl(2)-$CF_3$(5) | Smp.143-145 |

## 2. Formulierungsbeispiele (% = Gewichtsprozent)

### 2.1. Emulsions-Konzentrate

| | a) | b) | c) |
|---|---|---|---|
| Wirkstoff aus Tabelle 4 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus diesen Konzentraten können durch Verdünnen mit Wasser Emulsionen jeder gewünschten Konzentration hergestellt werden.

### 2.2. Emulsions-Konzentrate

| | a) | b) | c) |
|---|---|---|---|
| Wirkstoff aus Tabelle 4 | 10 % | 8 % | 60 % |
| Octylphenolpolyethylenglykolether (4-5 Mol Ethylenoxid) | 3 % | 3 % | 2 % |
| Ca-Dodecylbenzolsulfonat | 3 % | 4 % | 4 % |
| Ricinusölpolyglykolether (35 Mol Ethylenoxid) | 4 % | 5 % | 4 % |
| Cyclohexanon | 30 % | 40 % | 15 % |
| Xylolgemisch | 50 % | 40 % | 15 % |

Aus diesen Konzentraten können durch Verdünnen mit Wasser Emulsionen jeder gewünschten Konzentration hergestellt werden.

### 2.3. Suspensions-Konzentrat

| | |
|---|---|
| Wirkstoff aus Tabelle 4 | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |

| | |
|---|---|
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| <u>2.4. In Wasser dispergierbare Pulvermischungen</u> | a) | b) | c) |
|---|---|---|---|
| Wirkstoff aus Tabelle 4 | 25 % | 50 % | 75 % |
| Na-Ligninsulfonat | 5 % | 5 % | - |
| Oelsäure | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| <u>2.5. Stäubemittel</u> | a) | b) |
|---|---|---|
| Wirkstoff der Tabelle 4 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff und Vermahlen des Gemisches erhält man gebrauchsfertige Stäubemittel.

2.6. Granulat

| | a) | b) |
|---|---|---|
| Wirkstoff der Tabelle 4 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum eingedampft. Solche Granulate können dem Viehfutter beigemischt werden.

2.7. Granulat

| | |
|---|---|
| Wirkstoff aus Tabelle 4 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.8. Granulat

| | |
|---|---|
| Wirkstoff aus Tabelle 4 | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.9. Tabletten bzw. Boli

| | | |
|---|---|---|
| I | Wirkstoff der Tabelle 4 | 33,00 % |
| | Methylcellulose | 0,80 % |
| | Kieselsäure hochdispers | 0,80 % |
| | Maisstärke | 8,40 % |

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

I Methylcellulose wird in Wasser eingerührt. Nachdem das Material gequollen ist, wird Kieselsäure eingerührt und das Gemisch homogen suspendiert. Wirkstoff und Maisstärke werden gemischt. In diese Mischung wird die wässrige Suspension eingearbeitet und zu einem Teig geknetet. Die so erhaltene Masse wird durch ein 12 M-Sieb granuliert und getrocknet.

II Alle 4 Hilfsstoffe werden gut gemischt.

III Die gemäss I und II erhaltenen Vormischungen werden gemischt und zu Tabletten oder Boli verpresst.

## 3. Biologische Beispiele

Die anthelmintische Wirksamkeit wird anhand folgender Versuche demonstriert:

### 3.1. Versuch an mit Nematoden wie Haemonchus contortus und Trichostrongylus colubriformis infizierten Schafen

Der Wirkstoff wird in Form einer Suspension mit einer Magensonde oder durch Pansen-Injektion Schafen verabreicht, die vorher mit Nematoden wie Haemonchus contortus und Trichostrongylus colubriformis künstlich infiziert wurden. Pro Versuch resp. pro Dosis werden 1 bis 3 Tiere verwendet. Jedes Schaf wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen wird.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich.

In diesem Test wird mit Verbindungen der Formel I eine starke Reduzierung des Nematodenbefalls erzielt. So wird beispielsweise beim Einsatz von 20 mg Wirksubstanz pro kg Körpergewicht mit den nachfolgenden Verbindungen eine Reduzierung des Nematodenbefalls um mindestens 90 % bewirkt: 1.2, 1.5, 1.7, 1.9, 1.11, 1.13, 1.14, 1.15, 1.16, 1.19, 1.20, 1.21, 1.22, 1.23, 1.24, 1.27, 1.28, 1.29 und 1.30. Dieses Ergebnis wird bei einzelnen Verbindungen auch noch mit weiter herabgesetzter Dosis, beispielsweise mit 12 mg Wirksubstanz pro kg Körpergewicht oder noch geringeren Mengen an Wirksubstanz, erreicht.

3.2. Versuch an mit Cestoden wie Moniezia benedeni infizierten Schafen

Der Wirkstoff wird in Form einer Suspension mit einer Magensonde oder durch Pansen-Injektion Schafen verabreicht, die vorher mit Cestoden wie Moniezia benedeni artifiziell infiziert wurden. Pro Versuch resp. pro Dosis werden 3 Tiere verwendet. Jedes Schaf wird mit nur einer einzigen Dosis behandelt.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich. In diesem Versuch bewirken Wirkstoffe aus der Tabelle 4, wie beispielsweise die Verbindungen Nr. 1.15, 1.20 und 1.21 bei Dosen von weniger als 20 mg/kg Körpergewicht eine 90%ige Reduktion des Cestodenbefalls.

3.3. Versuch an mit Fasciola hepatica infizierten Schafen

Der Wirkstoff wird in Form einer Suspension mit einer Magensonde oder durch Pansen-Injektion Schafen verabreicht, die vorher mit Fasciola hepatica artifiziell infiziert wurden. Pro Versuch resp. pro Dosis werden 3 Tiere verwendet. Jedes Tier wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen wird.

Drei bis vier Wochen nach der Behandlung werden die Schafe getötet und seziert. Die Auswertung erfolgt durch das Auszählen der nach der Behandlung in den Gallengängen zurückgebliebenen Leberegel. Gleichzeitig und gleichartig infizierte aber unbehandelte Schafe dienen als Kontrolle resp. Vergleich. Der Unterschied der in den beiden Gruppen festgestellten Anzahl von Leberegeln ergibt den Wirkungsgrad des geprüften Wirkstoffs.

In diesem Versuch zeigen Wirkstoffe aus der Tabelle 4 bei Dosen von weniger als 20 mg/kg Körpergewicht eine mindestens 95%ige Wirksamkeit gegen Fasciola hepatica. Unter diesen Wirkstoffen ist die Verbindung Nr. 1.11 bei 12 mg/kg Körpergewicht voll wirksam gegen Fasciola hepatica.

Patentansprüche:

1. 5-Phenylcarbamoylthiobarbitursäurederivate der allgemeinen Formel I

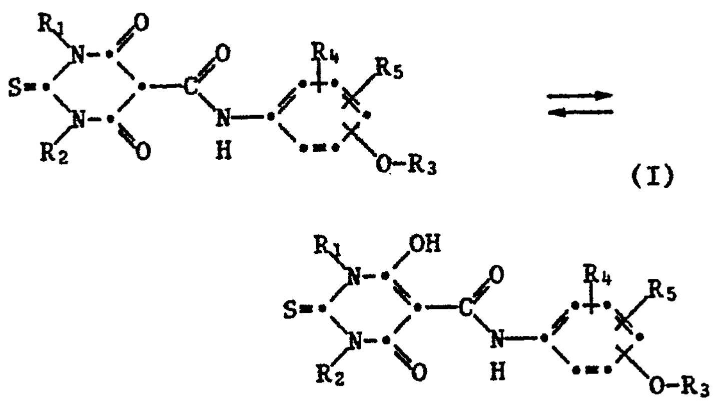

(I)

worin $R_1$ und $R_2$ unabhängig voneinander $C_1$-$C_5$-Alkyl oder Methoxy, $R_3$ unsubstituiertes Phenyl, unsubstituiertes Pyridyl, oder ein- bis dreifach durch $C_1$-$C_5$-Alkyl, Halogen, Nitro, $C_1$-$C_5$-Halogenalkyl mit 1 bis 5 Halogenatomen oder Cyano-$C_1$-$C_4$-Alkyl substituiertes Phenyl oder ein- bis dreifach durch $C_1$-$C_5$-Alkyl, Halogen, Nitro oder $C_1$-$C_5$-Halogenalkyl mit 1 bis 5 Halogenatomen substituiertes Pyridyl und $R_4$ und $R_5$ unabhängig voneinander Wasserstoff, Halogen, $C_1$-$C_5$-Alkyl, $C_1$-$C_2$-Halogenalkyl mit 1 bis 3 Halogenatomen, $C_1$-$C_3$-Alkoxy oder Nitro bedeuten, einschliesslich ihrer tautomeren Formen und Salze.

2. Verbindungen gemäss Anspruch 1, dadurch gekennzeichnet, dass $R_1$ und $R_2$ unabhängig voneinander $C_1$-$C_5$-Alkyl oder Methoxy, $R_3$ unsubstituiertes Phenyl, unsubstituiertes Pyridyl, oder ein- bis dreifach durch $C_1$-$C_5$-Alkyl, Halogen, Nitro, $C_1$-$C_5$-Halogenalkyl mit 1 bis 5 Halogenatomen oder Cyano-$C_1$-$C_4$-Alkyl substituiertes Phenyl oder ein- bis dreifach durch $C_1$-$C_5$-Alkyl, Halogen, Nitro oder $C_1$-$C_5$-Halogenalkyl mit 1 bis 5 Halogenatomen substituiertes Pyridyl und $R_4$ und $R_5$ unabhängig voneinander Wasserstoff, Halogen, $C_1$-$C_5$-Alkyl, $C_1$-$C_2$-Halogenalkyl mit 1 bis 3 Halogenatomen oder Nitro bedeuten.

3. Verbindungen gemäss Anspruch 1, dadurch gekennzeichnet, dass
$R_1$ Methyl, Aethyl oder Methoxy,
$R_2$ Methyl
$R_3$ unsubstituiertes Phenyl, unsubstituiertes Pyridyl oder ein- oder zweifach durch $C_1$-$C_5$-Alkyl, Halogen, Nitro, $C_1$-$C_5$-Halogenalkyl mit 1 bis 3 Halogenatomen oder Cyano-$C_1$-$C_4$-Alkyl substituiertes Phenyl oder ein- oder zweifach durch $C_1$-$C_5$-Alkyl, Halogen, Nitro oder $C_1$-$C_5$-Halogenalkyl mit 1 bis 3 Halogenatomen substituiertes Pyridyl und
$R_4$ und $R_5$ unabhängig voneinander Wasserstoff, $C_1$-$C_5$-Alkyl oder Halogen bedeuten.

4. Verbindungen gemäss Anspruch 1, dadurch gekennzeichnet, dass
$R_1$ Methyl oder Methoxy,
$R_2$ Methyl
$R_3$ ein durch Methyl, Chlor, Fluor, Trifluormethyl oder Cyanomethyl substituiertes Phenyl oder ein durch Methyl, Chlor, Fluor oder Trifluormethyl substituiertes Pyridyl und $R_4$ und $R_5$ unabhängig voneinander Wasserstoff, $C_1$-$C_3$-Alkyl, Chlor oder Nitro bedeuten.

5. Verbindungen gemäss Anspruch 1, dadurch gekennzeichnet, dass
$R_1$ Methyl oder Methoxy,
$R_2$ Methyl,
$R_3$ ein durch Methyl, Chlor, Trifluormethyl oder Cyanomethyl substituiertes Phenyl oder ein durch Methyl, Chlor oder Trifluormethyl substituiertes Pyridyl und $R_4$ und $R_5$ unabhängig voneinander Wasserstoff oder $C_1$-$C_3$-Alkyl bedeuten und der Rest -O-$R_3$ in 2- oder 4-Stellung steht.

6. Verbindungen gemäss Anspruch 1, dadurch gekennzeichnet, dass
$R_1$ Methyl oder Aethyl,
$R_2$ Methyl,
$R_3$ Phenyl, welches durch Substituenten der Gruppe Fluor, Chlor, Nitro, Trifluormethyl und Cyanomethyl mono- oder disubstituiert ist, oder 2-Pyridyl, welches durch Substituenten der Gruppe Fluor, Chlor, Nitro und Trifluormethyl mono- oder disubstituiert ist,

$R_4$ Wasserstoff, Chlor, Methyl, Isopropyl oder Methoxy, und
$R_5$ Wasserstoff, Chlor oder Methyl bedeuten, einschliesslich ihrer Triäthylaminsalze.

7. Verbindungen gemäss Anspruch 1, dadurch gekennzeichnet, dass
$R_1$ und $R_2$ jeweils für Methyl,
$R_3$ für Phenyl, welches durch Substituenten der Gruppe Chlor und Trifluormethyl mono- oder disubstituiert ist, oder 2-Pyridyl, welches durch Substituenten der Gruppe Chlor und Trifluormethyl mono- oder disubstituiert ist,
$R_4$ für Wasserstoff, Methyl, Isopropyl oder Methoxy und
$R_5$ für Wasserstoff oder Methyl stehen, wobei der Rest $-OR_3$ in meta- oder para-Position zum Stickstoffatom der Carbamoylgruppe steht, einschliesslich ihrer Triäthylaminsalze.

8. Verbindungen gemäss Anspruch 1, dadurch gekennzeichnet, dass
$R_1$ und $R_2$ jeweils für Methyl,
$R_3$ für Phenyl, welches durch Substituenten der Gruppe Chlor und Trifluormethyl mono- oder disubstituiert ist, oder 2-Pyridyl, welches durch Substituenten der Gruppe Chlor und Trifluormethyl mono- oder disubstituiert ist,
$R_4$ für Wasserstoff oder Methoxy und
$R_5$ für Wasserstoff stehen, wobei der Rest $-OR_3$ in meta- oder para-Position zum Stickstoffatom der Carbamoylgruppe steht.

9. Eine Verbindung der Formel I gemäss Anspruch 1, ausgewählt aus der Gruppe
1,3-Dimethyl-5-[2-isopropyl-4-(5-trifluormethyl-pyridyl-2-oxy)-phenylcarbamoyl]-2-thiobarbitursäure,
1,3-Dimethyl-5-[4-(5-trifluormethyl-pyridyl-2-oxy)-phenylcarbamoyl]-2-thiobarbitursäure-triäthylaminsalz,
1,3-Dimethyl-5-[4-(3-chlor-5-trifluormethyl-pyridyl-2-oxy)-phenylcarbamoyl]-2-thiobarbitursäure,
1,3-Dimethyl-5-[2,6-dimethyl-4-(5-trifluormethyl-pyridyl-2-oxy)-phenylcarbamoyl]-2-thiobarbitursäure und

1,3-Dimethyl-5-[4-(3,5-dichlor-pyridyl-2-oxy)-phenylcarbamoyl]-2-thiobarbitursäure.

10. 1,3-Dimethyl-5-[4-(5-trifluormethyl-pyridyl-2-oxy)-phenylcarbamoyl]-2-thiobarbitursäure gemäss Anspruch 1.

11. 1,3-Dimethyl-5-[4-(4-trifluormethyl-phenoxy)-phenylcarbamoyl]-2-thiobarbitursäure gemäss Anspruch 1.

12. 1,3-Dimethyl-5-[4-(3-trifluormethyl-phenoxy)-phenylcarbamoyl]-2-thiobarbitursäure gemäss Anspruch 1.

13. 1,3-Dimethyl-5-[4-methoxy-3-(2-chlor-4-trifluormethyl-phenoxy)-phenylcarbamoyl]-2-thiobarbitursäure gemäss Anspruch 1.

14. 1,3-Dimethyl-5-[3-methoxy-4-(2-chlor-4-trifluormethyl-phenoxy)-phenylcarbamoyl]-2-thiobarbitursäure gemäss Anspruch 1.

15. 1,3-Dimethyl-5-[4-methoxy-3-(3,5-dichlor-pyridyl-2-oxy)-phenylcarbamoyl]-2-thiobarbitursäure gemäss Anspruch 1.

16. Verbindungen der Formel II

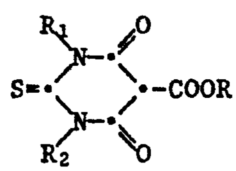

(II)

worin R $C_1$-$C_4$-Alkyl oder gegebenenfalls durch Nitro substituiertes Phenyl darstellt und $R_1$ und $R_2$ unabhängig voneinander $C_1$-$C_5$-Alkyl oder Methoxy bedeuten.

17. Verbindungen der Formel Va

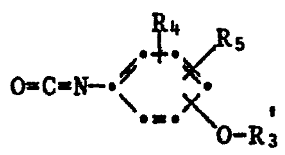

(Va)

worin $R'_3$ unsubstituiertes Pyridyl oder ein- bis dreifach durch $C_1$-$C_5$-Alkyl, Halogen, Nitro oder $C_1$-$C_5$-Halogenalkyl mit 1 bis 5 Halogenatomen substituiertes Pyridyl und $R_4$ und $R_5$ unabhängig voneinander Wasserstoff, Halogen, $C_1$-$C_5$-Alkyl, $C_1$-$C_2$-Halogenalkyl mit 1 bis 3 Halogenatomen, $C_1$-$C_3$-Alkoxy oder Nitro bedeuten.

18. Verfahren zur Herstellung der im Anspruch 1 beschriebenen Verbindungen der Formel I, dadurch gekennzeichnet, dass man

a) einen Ester der Formel II

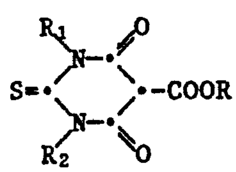

(II),

worin $R_1$ und $R_2$ unabhängig voneinander $C_1$-$C_5$-Alkyl oder Methoxy bedeuten, mit einem Anilinderivat der Formel III

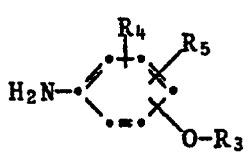

(III)

worin R ein $C_1$-$C_4$-Alkyl oder ein gegebenenfalls durch Nitro substituiertes Phenyl darstellt und

$R_3$ unsubstituiertes Phenyl, unsubstituiertes Pyridyl, oder ein- bis dreifach durch $C_1$-$C_5$-Alkyl, Halogen, Nitro, $C_1$-$C_5$-Halogenalkyl mit 1 bis 5 Halogenatomen oder Cyano-$C_1$-$C_4$-Alkyl substituiertes Phenyl oder ein- bis dreifach durch $C_1$-$C_5$-Alkyl, Halogen, Nitro oder $C_1$-$C_5$-Halogenalkyl mit 1 bis 5 Halogenatomen substituiertes Pyridyl und $R_4$ und $R_5$ unabhängig voneinander Wasserstoff, Halogen, $C_1$-$C_5$-Alkyl, $C_1$-$C_2$-Halogenalkyl mit 1 bis 3 Halogenatomen, $C_1$-$C_3$-Alkoxy oder Nitro bedeuten, bei Temperaturen von 50° bis 250°C umsetzt; oder

b) eine substituierte Thiobarbitursäure der Formel IV

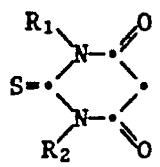

(IV)

worin $R_1$ und $R_2$ unabhängig voneinander $C_1$-$C_5$-Alkyl oder Methoxy bedeuten, mit einem substituierten Phenylisocyanat der Formel (V)

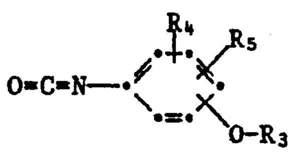

(V)

worin $R_3$ unsubstituiertes Phenyl, unsubstituiertes Pyridyl, oder ein- bis dreifach durch $C_1$-$C_5$-Alkyl, Halogen, Nitro, $C_1$-$C_5$-Halogenalkyl mit 1 bis 5 Halogenatomen oder Cyano-$C_1$-$C_4$-Alkyl substituiertes Phenyl oder ein- bis dreifach durch $C_1$-$C_5$-Alkyl, Halogen, Nitro oder $C_1$-$C_5$-Halogenalkyl mit 1 bis 5 Halogenatomen substituiertes Pyridyl und $R_4$ und $R_5$ unabhängig voneinander Wasserstoff, Halogen, $C_1$-$C_5$-Alkyl, $C_1$-$C_2$-Halogenalkyl mit 1 bis 3 Halogenatomen, $C_1$-$C_3$-Alkoxy oder Nitro bedeuten, bei Temperaturen von 0° bis 220°C umsetzt; oder

c) eine substituierte Thiobarbitursäure der Formel IV, worin $R_1$ und $R_2$ unabhängig voneinander $C_1$-$C_5$-Alkyl oder Methoxy bedeuten, mit einem substituierten Benzoylazid der Formel VI

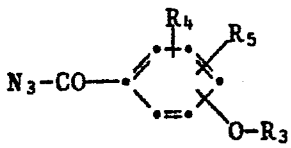

(VI)

worin $R_3$ unsubstituiertes Phenyl, unsubstituiertes Pyridyl, oder ein- bis dreifach durch $C_1$-$C_5$-Alkyl, Halogen, Nitro, $C_1$-$C_5$-Halogenalkyl mit 1 bis 5 Halogenatomen oder Cyano-$C_1$-$C_4$-Alkyl substituiertes Phenyl oder ein- bis dreifach durch $C_1$-$C_5$-Alkyl, Halogen, Nitro oder $C_1$-$C_5$-Halogenalkyl mit 1 bis 5 Halogenatomen substituiertes Pyridyl und $R_4$ und $R_5$ unabhängig voneinander Wasserstoff, Halogen, $C_1$-$C_5$-Alkyl, $C_1$-$C_2$-Halogenalkyl mit 1 bis 3 Halogenatomen, $C_1$-$C_3$-Alkoxy oder Nitro bedeuten, bei Temperaturen von 50° bis 250°C umsetzt.

19. Anthelmintisches Mittel, dadurch gekennzeichnet, dass es als aktive Komponente mindestens eine Verbindung der Formel I, ein Tautomeres oder ein Salz davon gemäss Anspruch 1 neben Träger- und weiteren Hilfsstoffen enthält.

20. Verfahren zur Bekämpfung parasitärer Helminthen, dadurch gekennzeichnet, dass man einem Tier eine anthelmintisch wirksame Menge einer Verbindung der Formel I gemäss Anspruch 1 verabreicht.

21. Verwendung einer Verbindung der Formel I gemäss Anspruch 1 zur Bekämpfung von parasitären Helminthen.

FO 7.5 SES/eg*